# EUROPEAN PATENT SPECIFICATION

(11) **EP 1 856 216 B1**
(45) Date of publication and mention of the grant of the patent: **16.07.2014**
(21) Application number: 06720808.2
(22) Date of filing: 16.02.2006
(51) Int. Cl.: C09D 11/00, C09D 163/00, C07D 317/36

(54) **SPRAYABLE COATING COMPOSITION**
SPRÜHBARE BESCHICHTUNGSZUSAMMENSETZUNG
PRÉPARATION POUR REVÊTEMENT VAPORISABLE

(30) Priority: 25.02.2005 GB 0503951
(43) Date of publication of application: 21.11.2007
(73) Proprietor: Sun Chemical Corporation, Parsippany, NJ 07054-1285 (US)
(72) Inventor: HERLIHY, Shaun Lawrence, Chatham, Kent ME5 9DD (GB); STANDING, Stephen Stuart, Orpington, Kent BR6 6BD (GB); DAVIDSON, Robert Stephen, Leicester LE5 2GG (GB)
(74) Representative: Schwahn, Hartmut
(86) International application number: PCT/US2006/005447
(87) International publication number: WO 2006/093680

(56) References cited:
- WO-A-02/42383
- WO-A-92/04383
- GB-A- 2 051 071
- US-A- 5 006 430
- US-A- 5 262 449
- PATENT ABSTRACTS OF JAPAN vol. 2000, no. 01, 31 January 2000 (2000-01-31) & JP 11 271972 A (NIPPON PAINT CO LTD), 8 October 1999 (1999-10-08)
- PATENT ABSTRACTS OF JAPAN vol. 1996, no. 05, 31 May 1996 (1996-05-31) & JP 08 020742 A (NIPPON KAYAKU CO LTD), 23 January 1996 (1996-01-23)

## Description

The present invention relates to new cationically curable sprayable coating compositions, such as printing inks or varnishes, having excellent cure and relatively low viscosities, as a result of the incorporation in the composition of unprecedentedly high levels of cyclic carbonates.

Although cationic curing of printing inks on exposure to ultraviolet radiation (UV) by the ring-opening polymerisation of epoxides has been known for a very long time, it has never achieved much commercial success, as a result, *inter alia,* of the slow cure speed of such systems. In order to make such systems commercially attractive, it is necessary to improve the cure speed of UV cationically curable epoxide-based printing inks and similar coating compositions.

We have surprisingly found that this may be achieved by the incorporation in the coating composition of relatively high levels of one or more cyclic carbonates, such as propylene carbonate. This finding is the more surprising, since propylene carbonate, in particular, is commonly used as a solvent for the cationic photoinitiator in such systems (the cationic photoinitiator commonly being used as a 50% solution in propylene carbonate) and since there is pressure from users of these coating compositions to reduce the level of propylene carbonate, on the basis that it may migrate out of the cured composition. Moreover, propylene carbonate is deemed by most formulators and end users to be an unreactive component, and so it would not be expected to have a positive effect on cure. Indeed, US Patent No. 5,262,449 is not alone in stating specifically that simple alkylene carbonates are merely solvents and play no part in polymerisation, and that they should be used in relatively low amounts to avoid undesired effects.

Since the level of propylene carbonate in prior art compositions is determined by the level of cationic photoinitiator, it is readily possible to determine the levels of propylene carbonate in the resulting compositions. In general, sulphonium salt cationic photoinitiators have been used in the prior art at levels of from 8 to 10% by weight, and so the level of propylene carbonate in such compositions would be from 4 to 5% by weight.

Carroy ["New Developments in Cationic Curing Flexo Inks", a paper presented at RadTech e/5 2004 Technical Proceedings] discloses a composition containing about 13.4% propylene carbonate, but attributes the results he achieved to the excellent thioxanthonium cationic photoinitiator which he used and its good dissolution in the printing ink.

JP 2004-32361 (Konica Minolta) also discloses a coating composition for ink jet use that contains either a cyclic ester compound (in an amount between 2.5 and 20 mass%, preferably between 5.0 and 15 mass%, of the total ink mass) or propylene carbonate (in unspecified amounts).

Moreover, for applications such as inkjet printing, which is becoming an increasingly important sector in the printing ink industry, it is necessary that the ink compositions should be of a relatively low viscosity. Similar requirements apply for other applications, such as paints or non-pigmented coatings for manufactured items, such as cars, where the composition needs to be sprayable, and it would be desirable to have the ability to spray and then cure by radiation, such as UV, rather than by stoving, which is commonly used at present. Other applications where a sprayable composition is desired include wood coatings and primers.

Not surprisingly, it has been difficult to formulate coatings for these types of applications that meet the combined requirements of high cure speed and low viscosity. However, we have now discovered that this may be achieved by the use of a cyclic carbonate in an amount higher than is conventional.

Thus, in one aspect, the present invention consists in a sprayable energy-curable coating composition comprising an epoxide monomer or oligomer, a cationic photoinitiator and a cyclic carbonate, the cyclic carbonate being present in an amount of at least 7% by weight of the entire composition.

In a further aspect, the present invention consists in a sprayable energy-curable coating composition comprising an epoxide monomer or oligomer, a cationic photoinitiator and a cyclic carbonate other than propylene carbonate.

In a still further aspect, the present invention consists in a sprayable energy-curable coating composition comprising an epoxide monomer or oligomer, a cationic photoinitiator and a cyclic carbonate, the cyclic carbonate being present in an amount of from 15% to 35% by weight of the entire composition.

We have also found that the compositions of the present invention are useful for rapid profiling (i.e. "printing" three dimensional objects) and so, in a yet further aspect, the present invention consists in a process for producing a three dimensional object by spraying a composition as defined above in a series of layers to build up the three dimensional object and curing the layers by exposing them to curing energy, e.g. UV.

Surprisingly, we have discovered that the use of a cyclic carbonate in an amount in excess of that previously used would lead to enhanced cure speed and post-cure. As a result of this, since the cyclic carbonates are generally liquid, and function as solvents, this has the effect of reducing the viscosity of the composition prior to curing, thus enabling the production of sprayable compositions which may then be cured by exposure to curing energy, e.g. electron beam or ultraviolet (UV).

Typical epoxides which may be used in the present invention include the cycloaliphatic epoxides (such as those sold under the designations Cyracure UVR6105, UVR6107, UVR6110 and UVR6128, by Dow), which are well known to those skilled in the art.

Other epoxides which may be used include such epoxy-functional oligomers/monomers as the glycidyl ethers of polyols [bisphenol A, alkyl diols or poly(alkylene oxides), which be di-, tri-, tetra- or hexa- functional]. Also, epoxides derived by the epoxidation of unsaturated materials may also be used (e.g. epoxidised soybean oil, epoxidised polybutadiene or epoxidised alkenes). Naturally occurring epoxides may also be used, including the crop oil collected from Vernonia galamensis.

The epoxides are one polymerisable species which are used in the compositions of the present invention. However, if desired, one or more other polymerisable species may also be included, for example an oxetane, which may be a mono-functional or multi-functional oxetane. These compounds are capable of polymerising by a cationically induced ring-opening reaction.

Examples of suitable mono-functional oxetanes include 3-ethyl-3-hydroxymethyl-oxetane or 3-ethyl-3-[2-ethylhexyloxy)methyl]oxetane. However, the compositions of the present invention are preferably free from added mono-functional oxetanes.

A variety of multi-functional oxetane compounds is available for use in the compositions of the present invention. For example, one such class of compounds are those compounds of formula (I): 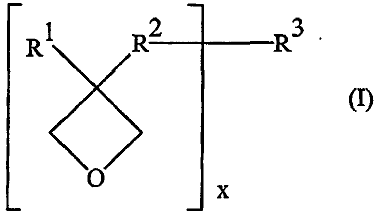 in which:
R¹ represents a hydrogen atom, a C₁ - C₆ alkyl group, an aryl group or an aralkyl group;
R² represents a direct bond or a C₁ - C₆ alkylene group;
R³ represents the residue of a polyol; and
x is a number from 2 to 6.

In the compounds of formula (I), x is more preferably from 2 to 4, still more preferably x is 2.

A further class of oxetane compounds which may be used in the compositions of the present invention are those compounds of formula (II): 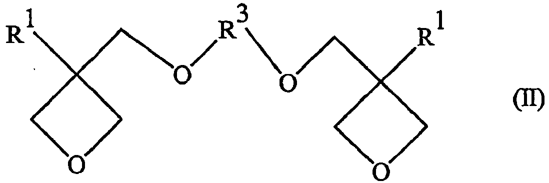 in which:
R¹ represents a hydrogen atom, a C₁ - C₆ alkyl group, an aryl group or an aralkyl group, and the two groups R¹ may be the same as or different from each other; and
R³ represents a C₁ - C₁₂ alkylene group, a C₂ - C₁₂ alkenylene group, a poly(alkyleneoxy) group, a carbonyl group, a C₂ - C₁₂ alkylene group in which a methylene group is replaced by a carbonyl group, an aryl group.

In the compounds of formula (II), we prefer that R³ should represent a C₁ - C₆ alkylene group.

A further class of oxetane compounds which may be used in the compositions of the present invention are those compounds of formula (III): 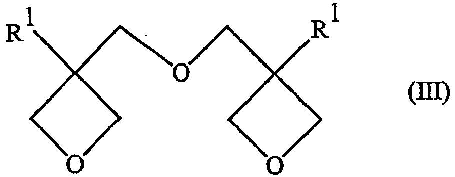 in which R¹ represents a hydrogen atom, a C₁ - C₆ alkyl group, an aryl group or an aralkyl group, and the two groups R¹ may be the same as or different from each other.

A particularly preferred example of the compounds of formula (III) is bis[(1-ethyl-3-oxetanyl)methyl] ether.

A further class of oxetane compounds which may be used in the compositions of the present invention are those compounds formula (IV): 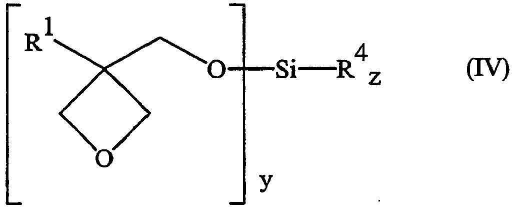 R¹ represents a hydrogen atom, a C₁ - C₆ alkyl group, an aryl group or an aralkyl group;
R⁴ represents a group of formula -O-R⁵ or a group R⁶;
R⁵ represents a C₁ - C₂₀ alkyl group, a C₂ - C₂₀ alkenyl group, an aryl group, an aralkyl group, a polyalkylene oxide group or a poly(lactone) group;
R⁶ represents a C₁ - C₂₀ alkyl group, an aryl group or an aralkyl group;
y is a number greater than 1 and no greater than 4; and
(y+z)=4.

Particularly preferred compounds of formula (IV) include the compounds of formula (V): 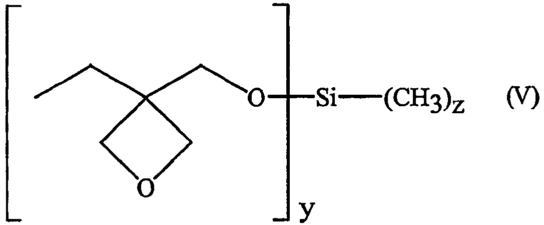 where y is a number of at least 2 and no greater than 4 and z is (4-y).

Compounds of formula (IV) and (V) are disclosed in GB 2393444, the disclosure of which is incorporated herein by reference.

Other examples of oxetane compounds which may be used in the present invention include compounds of formula (VI): 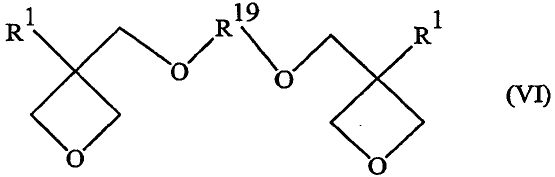 in which R¹⁹ represents a group of formula (VII), (VIII), or (IX) or a carbonyl group: 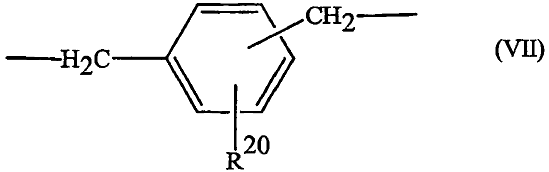 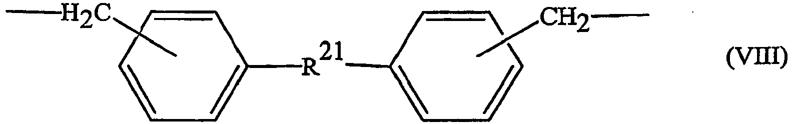 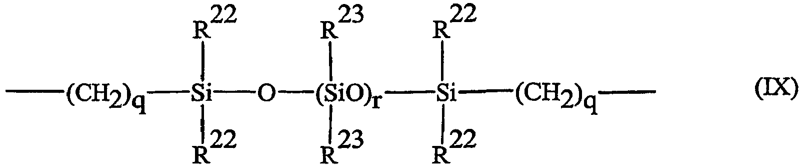 in which:
R²⁰ represents a C₁ - C₄ alkyl group (e.g. methyl, ethyl, propyl or butyl), a C₁ - C₄ alkoxy group (e.g. methoxy, ethoxy, propoxy or butoxy), a halogen atom (e.g. chlorine or bromine), a nitro group, a cyano group, a mercapto group, a C₁ - C₄ alkylthio group, a carboxy group, a C₂ - C₅ alkoxycarbonyl group or a carbamoyl group;
R²¹ represents an oxygen atom, a sulphur atom, a methylene group, or a group -SO-, -SO₂-, -C(CF₃)₂- or -C(CH₃)₂-;
q is a number from 1 to 6, preferably 3;
R²² represents a C₁ - C₄ alkyl group (e.g. methyl, ethyl, propyl or butyl) or an aryl group (e.g. phenyl);
r is a number from 0 to 2000; and
R²³ represents a C₁ - C₄ alkyl group (e.g. methyl, ethyl, propyl or butyl), an aryl group (e.g. phenyl) or a group of formula (X): 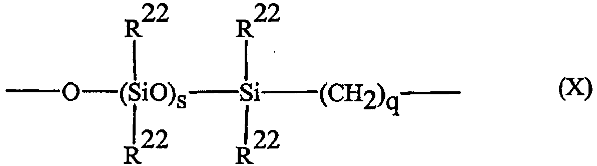 in which R²² and q are as defined above and s is a number from 0 to 100.

Preferred oxetanes are 3-ethyl-3-hydroxymethyl-oxetane, bis[(1-ethyl-3-oxetanyl)methyl] ether, 3-ethyl-3-[2-ethylhexyloxy)methyl]oxetane or [1,4-bis(3-ethyl-3-oxetanylmethoxy)methyl]benzene.

Another class of multi-functional oxetanes for use in the compositions of the present invention are linear polymers and copolymers having a plurality of oxetane groups, preferably an oxetane derivative of a novolac resin, for example the multifunctional oxetane sold as PNOX, available from Taogosei Co. Ltd.

As well as epoxides and optionally oxetanes, other reactive monomers/oligomers which may be used include the vinyl ethers of polyols [such as triethylene glycol divinyl ether, 1,4-cyclohexane dimethanol divinyl ether and the vinyl ethers of poly(alkylene oxides)]. Examples of vinyl ether functional prepolymers include the urethane-based products supplied by Allied Signal. Similarly, monomers/oligomers containing propenyl ether groups may be used in place of the corresponding compounds referred to above containing vinyl ether groups.

Other reactive species can include styrene derivatives and cyclic esters (such as lactones and their derivatives).

The composition of the present invention also contains a cationic photoinitiator. There is no particular restriction on the particular cationic photoinitiator used, and any cationic photoinitiator known in the art may be employed. Examples of such cationic photoinitiators include sulphonium salts (such as the mixture of compounds available under the trade name UVI6992 from Dow Chemical), thianthrenium salts (such as Esacure 1187 available from Lamberti), iodonium salts (such as IGM 440 from IGM) and phenacyl sulphonium salts. However, particularly preferred cationic photoinitiators are the thioxanthonium salts, such as those described in WO 03/072567 A1, WO 03/072568 A1, and WO 2004/055000 A1, the disclosures of which are incorporated herein by reference.

Particularly preferred thioxanthonium salts are those of formulae (XI), (XII) and (XIII):

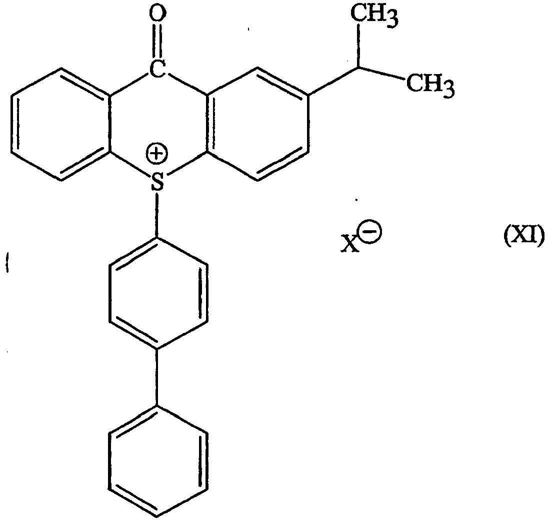

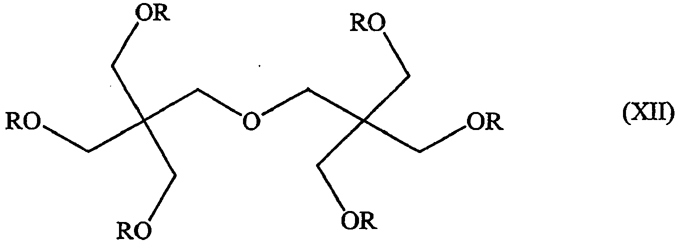

R-(OCH₂CH₂CH₂CH₂)ₙ-OR (XIII)

in which each R represents a group of formula (XIV): 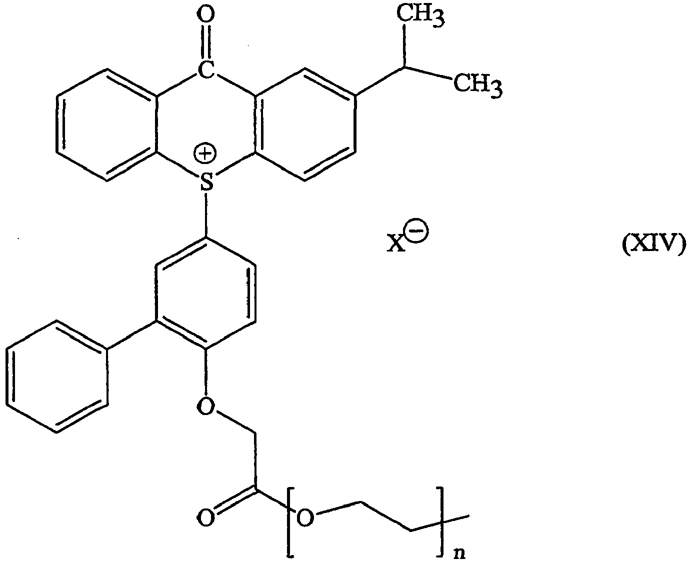 where n is a number and X⁻ is an anion, especially the hexafluorophosphates. The hexafluorophosphates of the compounds of formulae (I) and (II) are available from Robinson Brothers Ltd. under the trade marks "Meerkat" and "Bobcat", respectively, or from IGM under the trade marks IGM 550 and IGM 650 respectively.

The compositions of the present invention also contain a cyclic carbonate at a level higher than is conventionally used, when it is merely present as a solvent for the cationic photoinitiator, i.e. at a level of at least 7% by weight of the entire composition, preferably at least 8% by weight of the entire composition, more preferably at least 10% by weight of the entire composition, still more preferably at least 12% by weight of the entire composition, and most preferably at least 15% by weight of the entire composition. The amount of cyclic carbonate can go up to very high levels, far beyond what would previously have been considered sensible, even as far as 40% by weight of the entire composition, although, at such a level, its presence will tend to degrade the properties of the cured coating composition, and a more reasonable maximum is 35%, still more preferably 30%. In general, an amount of from 8% to 35% by weight of the entire composition is preferred, more preferably from 10% to 30% by weight of the entire composition, still more preferably from 12% to 25% by weight of the entire composition, and most preferably from 15% to 25% by weight of the entire composition.

The cyclic carbonate used may be any known in the art, preferably one that can act as a solvent for at least some part of the composition of the present invention prior to curing. Preferred cyclic carbonates are those having a 5-membered ring. Examples of suitable cyclic carbonates include compounds of formula (XV): 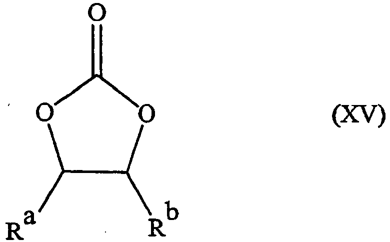 in which R^{a} and R^{b} are the same as or different from each other and each represents a hydrogen atom, a C₁ - C₃ alkyl group, a C₁ - C₃ hydroxyalkyl group or a C₂ - C₃ alkenyl group.

Where R^{a} and/or R^{b} represents an alkyl group, this may be, for example, a methyl, ethyl, propyl or isopropyl group, the methyl group being preferred. Where R^{a} and/or R^{b} represents a hydroxyalkyl group, this may be, for example, a hydroxymethyl, 1-hydroxyethyl, 2-hydroxyethyl, 2-hydroxypropyl or 3-hydroxypropyl group, the hydroxymethyl group being preferred. Where R^{a} and/or R^{b} represents an alkenyl group, this may be a vinyl or allyl group, the vinyl group being preferred.

Specific examples of such cyclic carbonates include propylene carbonate, glycerine carbonate, vinyl ethylene carbonate, ethylene carbonate and butylene carbonate, of which propylene carbonate is preferred.

In order to be sprayable, it is necessary that the viscosity of the composition of the present invention should be carefully controlled. Since a relatively high level of cyclic carbonate is included in the composition, it is not difficult to achieve a viscosity within the range which is sprayable in any particular application. However, it is necessary to avoid the inclusion in the composition of thickeners or materials which would tend to gel under the conditions employed. We prefer that the viscosity should be no greater than 50 cps at 25°C, and more preferably no greater than 35 cps at 25°C. In practice, we would also prefer that the viscosity should not be less than 7 cps at 25°C, and more preferably not less than 10 cps at 25°C. In particular, we prefer that the viscosity should be in the range of from 7 to 50 cps at 25°C, more preferably from 10 to 35 cps at 25°C.

However, for use as an ink jet ink in rapid profiling applications, a different viscosity profile is required. Specifically, for this use, we prefer that the ink a viscosity from 7 to 50 cps at an elevated jetting temperature of 50-80°C and a viscosity of greater than 200 cps at 25°C.

It is also common to include polyols in ultraviolet cationic curable formulations, which promote the cross-linking by a chain-transfer process. Examples of polyols include the ethoxylated/propoxylated derivatives of, for example, trimethylolpropane, pentaerythritol, di-trimethylolpropane, di-pentaerythritol and sorbitan esters, as well as more conventional poly(ethylene oxide)s and poly(propylene oxide)s. Other polyols well known to those skilled in the art are the polycaprolactone diols, triols and tetraols, such as those supplied by Dow.

Additives which may be used in conjunction with the principal components of the coating formulations of the present invention include stabilisers, plasticisers, pigments, waxes, slip aids, levelling aids, adhesion promoters, surfactants and fillers.

The amounts of the various components of the curable composition of the present invention may vary over a wide range and, in general, are not critical to the present invention. However, we prefer that the amount of the polymerisable components (i.e. the epoxide, oxetane, preferably multi-functional oxetane, if used, and other monomers, prepolymers and oligomers, if used) should be from 40 to 90%. The epoxide(s) preferably comprise from 30 to 80% of the polymerisable components in the composition of the present invention, and the multi-functional oxetane(s), if used, preferably comprise from 5 to 40% of the polymerisable components in the composition of the present invention. The amount of cationic photoinitiator is normally from 1.0 to 10% by weight, more preferably from 2.0 to 8%, by weight of the entire composition.

Other components of the curable composition may be included in amounts well known to those skilled in the art.

The composition of the present invention may be formulated as a printing ink, varnish, adhesive, paint, non-pigmented coating, wood coating, primer or any other coating composition which is intended to be cured by energy, which may be supplied by irradiation, whether by ultraviolet or electron beam. Such compositions will normally contain at least a polymerisable monomer, prepolymer or oligomer, and a cationic photoinitiator, as well as the cyclic carbonate, but may also include other components well known to those skilled in the art, for example, reactive diluents and, in the case of printing inks and paints, a pigment or dye.

The curable compositions of this invention may be suitable for applications that include protective, decorative and insulating coatings; potting compounds; sealants; adhesives; photoresists; textile coatings; and laminates. The compositions may be applied to a variety of substrates, e.g., metal, rubber, plastic, wood, moulded parts, films, paper, glass cloth, concrete, and ceramic. The curable compositions of this invention are particularly useful as inks for use in a variety of printing processes, including, but not limited to, flexography, inkjet and gravure. Details of such printing processes and of the properties of inks needed for them are well known and may be found, for example, in The Printing Ink Manual, 5th Edition, edited by R.H. Leach et al., published in 1993 by Blueprint, the disclosure of which is incorporated herein by reference.

Where the compositions of the present invention are used for inks, these typically comprise, as additional components to those referred to above, one or more of pigments, dyes, waxes, stabilisers, and flow aids, for example as described in "The Printing Ink Manual".

In particular, the compositions of the present invention are especially suitable for use in inkjet inks, especially for use in a drop on demand inkjet printhead.

They may be sold as a package in a container equipped with a spray nozzle (especially when intended for use as a paint or the like) or in an inkjet cartridge.

When used as inks, the compositions of the present invention show significant advantages over the prior art since to attain ink jet viscosities with cationic inks has previously required either high levels of vinyl ether, which results in slow cure, poor film strength and odour, or high levels of bis[(1-ethyl-3-oxetanyl)methyl] ether, which results in high formulation cost, and brittle films.

Both of these approaches also suffered from poor adhesion on some plastics since there was no attack of the ink in to the substrate (e.g. vinyl polymers).

The compositions of the present invention show benefits when used in various inkjet printing applications:
in line addressing/coding and card decoration (benefits from fast cure and superior adhesion);
wide format graphics (lower cure doses required means light weight lower power UV sources can be used);
single pass printing at high speed without the need for nitrogen blanketing used with acrylic formulations.

The invention also provides method of coating a substrate, in which a composition according to the present invention is sprayed onto the substrate and is then cured by exposure to curing energy.

The invention is further illustrated by the following non-limiting Examples. Percentages are by weight.

### EXAMPLE 1

A varnish formulation suitable for spray applications was prepared based on 2% Meerkat photoinitiator, 0.2% Tegorad 2100 wetting aid, 10% propylene carbonate, 40% OXT-221 dioxetane and 47.9% UVR6105 cycloaliphatic epoxide. This varnish had a measured viscosity of 16.5 seconds on a Ford 4 cup at 20°C.

The photoinitiator Meerkat (10-biphenyl-4-yl-2-isopropyl-9-oxo-9H-thioxanthen-10-ium hexafluorophosphate) was obtained from Robinson Brothers. Tegorad 2100 is a wetting aid obtained from the Tego Corporation. The cycloaliphatic epoxide resin UVR6105 (3,4-epoxycyclohexylmethyl 3,4-epoxycyclohexane carboxylate) was obtained from DOW. Propylene carbonate was obtained from Aldrich. The di-oxetane compound OXT-221, (bis[1-ethyl(3-oxetanyl)]methyl ether), was obtained from Toagosei.

The varnish was sprayed onto solvent washed uncoated steel panels using a Binks L600 gravity fed spray gun. The coated panels were then passed at 50 and 80 m/minute under a 300 W/inch medium pressure mercury arc lamp operating at full power. At both line speeds the coating cured with a single pass to give a glossy hard tack-free surface.

### EXAMPLE 2

Two varnish formulations suitable for inkjet application using a Piezo drop on demand inkjet head such as Spectra NOVA 256 or SL 128 print modules were prepared. These were printed onto 3 different substrates as a 12 micron thick film using a number 2 K bar;
- polycarbonate (Makrolon GP cIear 099 ex. BAYER)
- acrylic (plexiglass XT clear 20070 ex. Rohm & Haas)
- DiBond (Reynobond 33 white 903 ex. Alcoa)

The prints were cured under a 300 W/inch medium pressure mercury lamp at 42 m/minute. Viscosities were measured using a Brookfield DVII+ viscometer. The results are shown in Table 1.

**Table 1**

| | Formulation A | Formulation B |
|---|---|---|
| OXT-221 dioxetane | 40 | 20 |
| Meerkat photoinitiator | 2 | 2 |
| UVR 6105 cycloaliphatic epoxide | 46.9 | 61.9 |
| Propylene carbonate | 11 | 16 |
| Megaface F479 Fluoro surfactant | 0.1 | 0.1 |
| | | |
| Viscosity at 50°C | 13.0 | 13.9 |

- Megaface F479 is a Fluoro surfactant ex Dianippon Ink and Chemical Co. Ltd.

Both varnishes cured well and gave an initial MEK rub resistance of greater than 50. There was no discernable odour on cure.

### EXAMPLE 3

Cyan ink formulations suitable for inkjet printing were prepared based on;

| | |
|---|---|
| 2.5% | Meerkat photoinitiator |
| 2.1 % | Sunfast Blue 249-3054 pigment |
| 0.1% | Megaface F479 Fluoro surfactant |
| 45.5% | UVR6105 cycloaliphatic epoxide |
| 29.8 - 49.8% | OXT-221 di-oxetane monomer ex Toagosei |
| 0-20% | propylene carbonate |

All formulations were formulated to a viscosity suitable for a Piezo drop on demand inkjet head such as Spectra NOVA 256 or SL 128 print modules. Formulations were printed at 12 microns thickness onto polycarbonate substrate (Makrolon GP cIear 099 ex. BAYER) using a number 2 K bar and were cured with a single pass under a 300 W/inch medium pressure mercury lamp at 42 m/minute. Cure was assessed using the well known MEK solvent rub method 30 seconds and 15 minutes after cure. Viscosities were measured using a Brookfield DVII+ viscometer. The results are shown in Table 2.

**Table 2**

| % propylene carbonate | Weight ratio UVR6105: propylene carbonate | Viscosity@ 50 °C cPs | MEK double rubs | |
|---|---|---|---|---|
| | | | T = 30 seconds | T= 15 minutes |
| 0 | - | 15.7 | 6 | 37 |
| 2.5 | 18.2: 1 | 15.2 | 85 | >200 |
| 5 | 9.1: 1 | 14.7 | 156 | >200 |
| 7.5 | 6.1: 1 | 14.0 | 158 | >200 |
| 10 | 4.6: 1 | 13.4 | >200 | >200 |
| 12.5 | 3.6: 1 | 12.6 | 130 | >200 |
| 15 | 3.0: 1 | 12.1 | 79 | 147 |
| 17.5 | 2.6: 1 | 11.8 | 55 | 75 |
| 20 | 2.3: 1 | 11.3 | 46 | 58 |

These results in Cyan inkjet formulations demonstrate that, for formulations based on cycloaliphatic epoxide, propylene carbonate and dioxetane monomer, the optimum cure efficiency is obtained at a level of approximately 10.0% by weight of propylene carbonate, corresponding to a weight ratio of 4.6 UVR6105 : 1 propylene carbonate. The inkjet formulations of this Example all cured substantially faster than equivalent UV curing free radical based inks.

### EXAMPLE 4

### Preparation of ink jet inks

The inkjet inks shown in Table 3 were made and tested in a piezo drop on demand print head. Specifically the inks were printed through a Spectra NOVA 256 print head fitted with Miata reservoir. The fire pulse voltage and jetting temperature were adjusted to obtain the correct nominal drop mass (80ng). Print sustainability was verified by printing at 100% duty cycle for 5 minutes at 2-12 kHz.

**Table 3**

| | **Black** | **Black** | **Black** | **Cyan** | **Cyan** | **Magenta** | **Yellow** |
|---|---|---|---|---|---|---|---|
| UVR 6105 cycloaliphatic epoxide | 28.0 | 41.2 | 46.2 | 31.9 | 40.9 | 33.8 | 31.8 |
| Dioxetane OXT 221 | 27.5 | 10.0 | | 28.3 | | | |
| Propylene Carbonate | 10.3 | 15.4 | 20.4 | 11.9 | 25.7 | 32.8 | 30.8 |
| Oxetane OXT 101 | | 10.0 | 10.0 | | 10.0 | 10.0 | 10.0 |
| IGM BL-550 Photo-initiator | 18.8 | 8.0 | 8.0 | 12.5 | 8.0 | 8.0 | 8.0 |
| Megaface F479 Fluoronated wetting agent | 0.4 | 0.4 | 0.4 | 0.4 | 0.4 | 0.4 | 0.4 |
| Violet 19 Dispersion | | | | | | 15.0 | |
| Yellow 150 Dispersion | | | | | | | 19.0 |
| Blue 15:3 Dispersion | | | | 15.0 | 15.0 | | |
| Black 7 Dispersion | 15.0 | 15.0 | 15.0 | | | | |
| **Total %** | **100.0** | **100.0** | **100.0** | **100.0** | **100.0** | **100.0** | **100.0** |
| Cure (mj/cm2) | 50 | 55 | 85 | 70 | 110 | 700 | 800 |
| **Viscosity @ 50°C** | **12.7 cP** | **12.5 cP** | **12.3 cP** | **12.9 cP** | **12.1 cP** | **12.0 cP** | **11.9 cP** |

| | | | | | | | |
|---|---|---|---|---|---|---|---|
| Omnicat BL-550 is a solution of 20% Omnicat 550 photoinitiator in 25% propylene carbonate and 55% of cycloaliphatic epoxide Cure - for 12 micron layer. 300W/inch un-doped medium pressure mercury lamp (Fusion F300) | | | | | | | |

### EXAMPLE 5

### Hot Melt Cationic Formulation

The composition shown in Table 4 was jetted through a Spectra Nova 256 80ng print head with Miata reservoir, running at a print head temperature of 75°C to form a layer. The composition was cured by LED exposure at 395nm (Phoseon RX10 unit) immediately after each layer of ink was layed down. The operation was repeated until the desired three dimensional object had been built up.

The ink composition was a low viscosity jetable liquid at 75°C but exists as a paste at room temperature.

**Table 4**

| | | |
|---|---|---|
| Epoxide 6105 | | 30.3 |
| Dioxetane | | 26.5 |
| Propylene Carbonate | | 11.9 |
| IGM BL-550 (CH052102) | | 12.5 |
| ITX | | 2.0 |
| Megaface F479 | | 0.4 |
| Croda Synchrowax HGLC | | 1.5 |
| Cyan Pigment dispersion | | 15.0 |
| | | **100.0** |
| | Viscosity @ 50°C | 12.8 cP |
| | S.T (dynes/cm²) | 26.0 |

## Claims

1. A sprayable energy-curable coating composition comprising an epoxide monomer or oligomer, a cationic photoinitiator and a cyclic carbonate, wherein the cyclic carbonate is 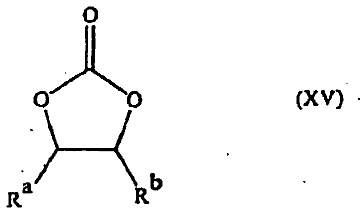 in which R^{a} and R^{b} are the same or different from each other and each represents hydrogen, a C₁-C₃ alkyl group, a C₁-C₃ hydroxyalkyl group or a C₁-C3 alkenyl group and when the cyclic carbonate is propylene carbonate, it is present in an amount of from 7% to 35% by weight of the entire composition.

2. A composition according to Claim 1, in which the cyclic carbonate is present in an amount of at least 8% by weight of the entire composition.

3. A composition according to Claim 2, in which the cyclic carbonate is present in an amount of at least 10% by weight of the entire composition.

4. A composition according to Claim 3, in which the cyclic carbonate is present in an amount of at least 12% by weight of the entire composition.

5. A composition according to Claim 4, in which the cyclic carbonate is present in an amount of at least 15% by weight of the entire composition.

6. A composition according to Claim 3, in which the cyclic carbonate is present in an amount of from 8% to 35% by weight of the entire composition.

7. A composition according to Claim 6 in which the cyclic carbonate is present in an-amount of from 10% to 30% by weight of the entire composition.

8. A composition according to Claim 7 in which the cyclic carbonate is present in an amount of from 12% to 25% by weight of the entire composition.

9. A composition according to Claim 8 in which the cyclic carbonate is present in an amount of from 15% to 25% by weight of the entire composition.

10. A composition according to Claim 6 in which the cyclic carbonate is present in an amount of from 15% to 35% by weight of the entire composition.

11. A composition according to any one of the preceding Claims, additionally comprising an oxetane monomer.

12. composition according to Claim 11 in which the oxetane is 3-ethyl-3-hydroxymethyl-oxetane, bis[(1-ethyl-3-oxetanyl)methyl] ether, 3-ethyl-3-[(2-ethylhexyloxy)methyl]oxetane or [1,4-bis(3-ethyl-3-oxetanylmethoxy)methyl]benzene.

13. A composition according to Claim 11, in which said oxetane is a linear polymer or copolymer having a plurality of oxetane groups.

14. A composition according to Claim 13, in which said oxetane is an oxetane derivative of an epoxy novolac resin.

15. A composition according to any one of the preceding Claims, in which the cyclic carbonate is propylene carbonate, glycerine carbonate, vinyl ethylene carbonate, ethylene carbonate or butylene carbonate.

16. A composition according to Claim 15, in which the cyclic carbonate is propylene carbonate.

17. A composition according to any one of Claims 1 to 14, in which the cyclic carbonate is a compound other than propylene carbonate.

18. A composition according to Claims 17, in which the cyclic carbonate is glycerine carbonate, vinyl ethylene carbonate, ethylene carbonate or butylene carbonate.

19. A composition according to any one of the preceding Claims, having a viscosity from 7 to 50 cps at 25°C.

20. A composition according to Claim 19, in which the viscosity is from 10 to 35 cps at 25°C.

21. A composition according to any one of the preceding Claims, in the form of a printing ink or varnish.

22. A composition according to any one of the preceding Claims, formulated for inkjet printing.

23. A composition according to Claim 19, which is suitable for use in a drop on demand inkjet printhead.

24. A composition according to Claim 22 or Claim 23, having a viscosity from 7 to 50 cps at an elevated jetting temperature of 50-80°C and a viscosity of greater than 200 cps at 25°C.

25. A composition according to any one of Claims 1 to 20, formulated as a paint.

26. A process for preparing a cured coating composition, which comprises applying a composition according to any one of the preceding Claims to a substrate and exposing the coated substrate to curing radiation sufficient to cure the coating.

27. A process according to Claim 26, in which the curing radiation is ultraviolet.

28. A package comprising a composition according to any one of Claims 1 to 21 in a container equipped with a spray nozzle.

29. A package comprising a composition according to any one of Claims 1 to 24 in an inkjet cartridge.

30. A method of coating a substrate, in which a composition according to any one of Claims 1 to 25 is sprayed onto the substrate and is then cured by exposure to curing energy.

31. A method according to Claim 30, in which the curing energy is ultraviolet or electron beam.

32. A process for producing a three dimensional object by spraying a composition according to any one of Claims 1 to 25 in a series of layers to build up the three dimensional object and curing the layers by exposing them to curing energy.

33. A process according to Claim 32, in which the curing energy is ultraviolet.

## Patentansprüche

1. Versprühbare, energiehärtbare Beschichtungszusammensetzung, aufweisend ein Epoxidmonomer oder -oligomer, einen kationischen Fotoinitiator und ein zyklisches Carbonat, wobei es sich bei dem zyklischen Carbonat um 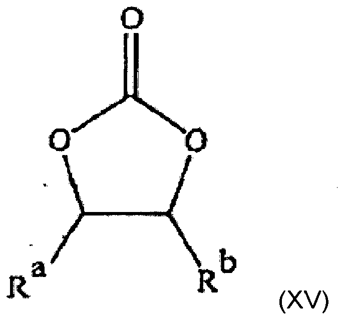 handelt,
wobei R^{a} und R^{b} identisch oder unterschiedlich voneinander sind und jeweils Wasserstoff, eine C₁-C₃-Alkylgruppe, eine C₁-C₃-Hydroxyalkylgruppe oder eine C₁-C₃-Alkenylgruppe darstellen und, wenn es sich bei dem zyklischen Carbonat um Propylencarbonat handelt, dieses in einer Menge von 7 Gew.% bis 35 Gew.% der gesamten Zusammensetzung vorhanden ist.

2. Zusammensetzung nach Anspruch 1, wobei das zyklische Carbonat in einer Menge von mindestens 8 Gew.% der gesamten Zusammensetzung vorhanden ist.

3. Zusammensetzung nach Anspruch 2, wobei das zyklische Carbonat in einer Menge von mindestens 10 Gew.% der gesamten Zusammensetzung vorhanden ist.

4. Zusammensetzung nach Anspruch 3, wobei das zyklische Carbonat in einer Menge von mindestens 12 Gew.% der gesamten Zusammensetzung vorhanden ist.

5. Zusammensetzung nach Anspruch 4, wobei das zyklische Carbonat in einer Menge von mindestens 15 Gew.% der gesamten Zusammensetzung vorhanden ist.

6. Zusammensetzung nach Anspruch 3, wobei das zyklische Carbonat in einer Menge von 8 Gew.% bis 35 Gew.% der gesamten Zusammensetzung vorhanden ist.

7. Zusammensetzung nach Anspruch 6, wobei das zyklische Carbonat in einer Menge von 10 Gew.% bis 30 Gew.% der gesamten Zusammensetzung vorhanden ist.

8. Zusammensetzung nach Anspruch 7, wobei das zyklische Carbonat in einer Menge von 12 Gew.% bis 25 Gew.% der gesamten Zusammensetzung vorhanden ist.

9. Zusammensetzung nach Anspruch 8, wobei das zyklische Carbonat in einer Menge von 15 Gew.% bis 25 Gew.% der gesamten Zusammensetzung vorhanden ist.

10. Zusammensetzung nach Anspruch 6, wobei das zyklische Carbonat in einer Menge von 15 Gew.% bis 35 Gew.% der gesamten Zusammensetzung vorhanden ist.

11. Zusammensetzung nach einem der vorhergehenden Ansprüche, die darüber hinaus ein Oxetanmonomer umfasst.

12. Zusammensetzung nach Anspruch 11, wobei es sich bei dem Oxetan um 3-Ethyl-3-hydroxymethyl-oxetan, Bis[(1-ethyl-3-oxetanyl)methyl]ether, 3-Ethyl-3-[(2-ethylhexyloxy)methyl]oxetan oder [1,4-bis(3-ethyl-3-oxetanylmethoxy)methyl]benzol handelt.

13. Zusammensetzung nach Anspruch 11, wobei es sich bei dem Oxetan um ein lineares Polymer oder Copolymer mit einer Mehrzahl von Oxetangruppen handelt.

14. Zusammensetzung nach Anspruch 13, wobei es sich bei dem Oxetan um ein Oxetanderivat eines Epoxid-Novolak-Harzes handelt.

15. Zusammensetzung nach einem der vorhergehenden Ansprüche, wobei es sich bei dem zyklischen Carbonat um Propylencarbonat, Glycerincarbonat, Vinylethylencarbonat, Ethylencarbonat oder Butylencarbonat handelt.

16. Zusammensetzung nach Anspruch 15, wobei es sich bei dem zyklischen Carbonat um Propylencarbonat handelt.

17. Zusammensetzung nach einem der Ansprüche 1 bis 14, wobei es sich bei dem zyklischen Carbonat um eine andere Verbindung als Propylencarbonat handelt.

18. Zusammensetzung nach Anspruch 17, wobei es sich bei dem zyklischen Carbonat um Glycerincarbonat, Vinylethylencarbonat, Ethylencarbonat oder Butylencarbonat handelt.

19. Zusammensetzung nach einem der vorhergehenden Ansprüche mit einer Viskosität von 7 bis 50 cps bei 25°C.

20. Zusammensetzung nach Anspruch 19, wobei die Viskosität bei 25°C von 10 bis 35 cps beträgt.

21. Zusammensetzung nach einem der vorhergehenden Ansprüche in Form einer Drucktinte oder eines Lacks.

22. Zusammensetzung nach einem der vorhergehenden Ansprüche, die zum Tintenstrahldrucken formuliert ist.

23. Zusammensetzung nach Anspruch 19, die zur Verwendung in einem Drop-on-Demand-Tintendruckkopf geeignet ist.

24. Zusammensetzung nach Anspruch 22 oder Anspruch 23 mit einer Viskosität von 7 bis 50 cps bei einer erhöhten Auftragstemperatur von 50 bis 80°C und einer Viskosität von über 200 cps bei 25°C.

25. Zusammensetzung nach einem der Ansprüche 1 bis 20, die als eine Anstrichfarbe formuliert ist.

26. Verfahren zur Zubereitung einer ausgehärteten Beschichtungszusammensetzung, das das Auftragen einer Zusammensetzung nach einem der vorhergehenden Ansprüche auf ein Substrat und das Bestrahlen des beschichteten Substrats mit genügend aushärtender Strahlung zur Aushärtung der Beschichtung aufweist.

27. Verfahren nach Anspruch 26, wobei die aushärtende Strahlung ultraviolett ist.

28. Paket aufweisend eine Zusammensetzung nach einem der Ansprüche 1 bis 21 in einem Behälter, der mit einer Sprühdüse ausgestattet ist.

29. Paket aufweisend eine Zusammensetzung nach einem der Ansprüche 1 bis 24 in einer Tintenstrahlpatrone.

30. Verfahren zur Beschichtung eines Substrats, wobei eine Zusammensetzung nach einem der Ansprüche 1 bis 25 auf das Substrat gesprüht wird und anschließend durch Bestrahlen mit einer aushärtenden Energie ausgehärtet wird.

31. Verfahren nach Anspruch 30, wobei die aushärtende Energie ultraviolett oder ein Elektronenstrahl ist.

32. Prozess zur Herstellung eines dreidimensionalen Objekts durch Versprühen einer Zusammensetzung nach einem der Ansprüche 1 bis 25 in einer Reihe von Schichten, um das dreidimensionale Objekt aufzubauen, und Aushärten der Schichten, indem diese Schichten einer aushärtenden Energie ausgesetzt werden.

33. Prozess nach Anspruch 32, in dem die aushärtende Energie ultraviolett ist.

## Revendications

1. Composition de revêtement pulvérisable et durcissable par énergie comprenant un monomère ou un oligomère de type époxyde, un photoinitiateur cationique et un carbonate cyclique, dans laquelle le carbonate cyclique est : 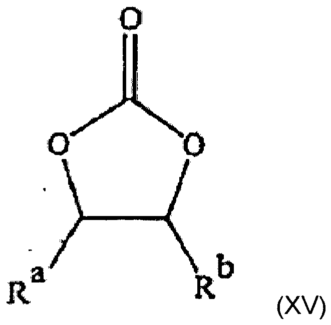 dans laquelle R^{a} et R^{b} sont identiques ou différents l'un de l'autre et chacun représente un atome d'hydrogène, un groupe alkyle de C₁ à C₃, un groupe hydroxyalkyle de C₁ à C₃ ou un groupe alcényle de C₁ à C₃ et, lorsque le carbonate cyclique est du carbonate de propylène, il est présent en une quantité de 7 % à 35 % en poids de la composition entière.

2. Composition selon la revendication 1, dans laquelle le carbonate cyclique est présent en une quantité d'au moins 8 % en poids de la composition entière.

3. Composition selon la revendication 2, dans laquelle le carbonate cyclique est présent en une quantité d'au moins 10 % en poids de la composition entière.

4. Composition selon la revendication 3, dans laquelle le carbonate cyclique est présent en une quantité d'au moins 12 % en poids de la composition entière.

5. Composition selon la revendication 4, dans laquelle le carbonate cyclique est présent en une quantité d'au moins 15 % en poids de la composition entière.

6. Composition selon la revendication 3, dans laquelle le carbonate cyclique est présent en une quantité de 8 % à 35 % en poids de la composition entière.

7. Composition selon la revendication 6, dans laquelle le carbonate cyclique est présent en une quantité de 10 % à 30 % en poids de la composition entière.

8. Composition selon la revendication 7, dans laquelle le carbonate cyclique est présent en une quantité de 12 % à 25 % en poids de la composition entière.

9. Composition selon la revendication 8, dans laquelle le carbonate cyclique est présent en une quantité de 15 % à 25 % en poids de la composition entière.

10. Composition selon la revendication 6, dans laquelle le carbonate cyclique est présent en une quantité de 15 % à 35 % en poids de la composition entière.

11. Composition selon l'une quelconque des revendications précédentes, comprenant en outre un monomère d'oxétanne.

12. Composition selon la revendication 11, dans laquelle l'oxétanne est le 3-éthyl-3-hydroxyméthyl-oxétanne, le bis[(1-éthyl-3-oxétanyl)méthyl]éther, le 3-éthyl-3-[(2-éthylhexyloxy)méthyl]oxétanne ou le [1,4-bis(3-éthyl-3-oxétanylméthoxy)méthyl]benzène.

13. Composition selon la revendication 11, dans laquelle ledit oxétanne est un polymère ou un copolymère linéaire qui comporte une pluralité de groupes oxétanne.

14. Composition selon la revendication 13, dans laquelle ledit oxétanne est un dérivé oxétanne d'une résine époxy novolaque.

15. Composition selon l'une quelconque des revendications précédentes, dans laquelle le carbonate cyclique est le carbonate de propylène, le carbonate de glycérine, le carbonate d'éthylène vinylique, le carbonate d'éthylène ou le carbonate de butylène.

16. Composition selon la revendication 15, dans laquelle le carbonate cyclique est le carbonate de propylène.

17. Composition selon l'une quelconque des revendications 1 à 14, dans laquelle le carbonate cyclique est un composé autre que le carbonate de propylène.

18. Composition selon la revendication 17, dans laquelle le carbonate cyclique est le carbonate de glycérine, le carbonate d'éthylène vinylique, le carbonate d'éthylène ou le carbonate de butylène.

19. Composition selon l'une quelconque des revendications précédentes, dont la viscosité varie de 7 à 50 cps à 25°C.

20. Composition selon la revendication 19, dans laquelle la viscosité varie de 10 à 35 cps à 25°C.

21. Composition selon l'une quelconque des revendications précédentes, sous la forme d'une encre ou d'un vernis d'impression.

22. Composition selon l'une quelconque des revendications précédentes, formulée pour une impression à jet d'encre.

23. Composition selon la revendication 19, laquelle est appropriée pour utilisation dans une tête d'impression à jet d'encre goutte à la demande.

24. Composition selon la revendication 22 ou la revendication 23, dont la viscosité varie de 7 à 50 cps à une température de jet élevée de 50 à 80°C et dont la viscosité est supérieure à 200 cps à 25°C.

25. Composition selon l'une quelconque des revendications 1 à 20, formulée comme une peinture.

26. Procédé pour la préparation d'une composition de revêtement durcie, lequel comprend l'application d'une composition selon l'une quelconque des revendications précédentes à un substrat et l'exposition du substrat enduit à un rayonnement de durcissement suffisant pour durcir le revêtement.

27. Procédé selon la revendication 26, dans lequel le rayonnement de durcissement est un rayonnement ultraviolet.

28. Emballage comprenant une composition selon l'une quelconque des revendications 1 à 21 dans un contenant doté d'une buse de pulvérisation.

29. Emballage comprenant une composition selon l'une quelconque des revendications 1 à 24 dans une cartouche pour jet d'encre.

30. Procédé de revêtement d'un substrat, dans lequel une composition selon l'une quelconque des revendications 1 à 25 est pulvérisée sur le substrat et est ensuite durci par exposition à une énergie de durcissement.

31. Procédé selon la revendication 30, dans lequel l'énergie de durcissement est un rayonnement ultraviolet ou un faisceau électronique.

32. Procédé pour la production d'un objet tridimensionnel par pulvérisation d'une composition selon l'une quelconque des revendications 1 à 25 dans une série de couches pour construire l'objet tridimensionnel et durcir les couches en les exposant à une énergie de durcissement.

33. Procédé selon la revendication 32, dans lequel l'énergie de durcissement est un rayonnement ultraviolet.
